# EUROPEAN PATENT SPECIFICATION

(11) **EP 2 427 149 B1**
(45) Date of publication and mention of the grant of the patent: **10.08.2016**
(21) Application number: 10719371.6
(22) Date of filing: 10.05.2010
(51) Int. Cl.: A61F 2/89, A61F 2/90, A61F 2/82, A61F 2/30

(54) **A MEDICAL DEVICE SUITABLE FOR LOCATION IN BODY LUMEN**
MEDIZINISCHE VORRICHTUNG, DIE ZUR ANORDNUNG IN EINEM KÖRPERHOHLRAUM GEEIGNET IST
DISPOSITIF MÉDICAL ADAPTÉ POUR LA MISE EN PLACE DANS UNE LUMIÈRE DU CORPS

(30) Priority: 08.05.2009 US 463039; 08.05.2009 EP 09251283
(43) Date of publication of application: 14.03.2012
(73) Proprietor: Veryan Medical Limited, Oxford OX4 2HN (GB)
(72) Inventor: TAYLOR, Charles, West Sussex RH13 6QR (GB); HERATY, Kevin, Co. Galway (IE); MULLINS, Liam, Galway (IE)
(74) Representative: Magrath, Sally Ann Mason
(86) International application number: PCT/GB2010/000944
(87) International publication number: WO 2010/128311

(56) References cited:
- EP-A1- 0 800 801
- EP-A1- 2 174 623
- EP-A1- 2 174 624
- WO-A1-98/20810
- WO-A1-2007/053791
- WO-A1-2008/125842
- GB-A- 2 418 362
- US-A- 5 938 697

## Description

This invention relates to a stent suitable for deployment in a blood vessel.

It is known from GB 2418362 (on which the pre-characterising part of claim 1 is based) to provide a stent having a three-dimensional curvature when deployed in a vessel.

It is known from WO 2007/053791 to provide a stent the expansion of which causes it to bend and attain a curved configuration corresponding to the curved vessel into which it is deployed. The bend in the stent may include a three-dimensional curve.

It is known from WO 98/20810 to provide a stent sufficiently flexible to bend and conform to a tortuous body lumen at the target site.

WO 2008/125842 discloses a method of making a helical graft from living tissue, the graft having a three-dimensional curvature.

US 5938697 discloses a stent exhibiting a varying outward radial force along its length.

EP 2174623 and EP 2174624 are patent applications falling within the terms of Art. 54 (3) EPC. EP 2174623 describes a stent movable between a collapsed delivery configuration and an expanded deployment configuration. In the delivery configuration the longitudinal axis of the central section is substantially straight, while in the deployment configuration it is curved in the three-dimensional space. EP 2174624 describes stents having an unloaded configuration which is intermediate between an unloaded and a loaded state of the blood vessel wherein the stent is to be deployed. The unloaded configuration of the stent can be straight or curved in a two-dimensional shape or curved in a three-dimensional shape.

### Statements of Invention

According to the invention there is provided a stent suitable for deployment in a blood vessel, wherein the stent is expandable from a delivery configuration to a deployment configuration, wherein upon application of a load to the stent when the stent is in the deployment configuration the stent is movable from an unloaded configuration to a loaded configuration, and wherein in the loaded configuration at least part of the longitudinal axis of the stent is curved in three-dimensional space, characterised in that in the unloaded configuration said part of the longitudinal axis of the stent is substantially straight or is curved in a two-dimensional plane, the stent being biased to achieve the three-dimensional curvature during loading.

The three-dimensional curved shape of the stent maximises the fracture resistance of the stent. In the case of certain types of loading, for example compressive loading, the three-dimensional curved shape of the stent may minimise points of stress concentration.

In the loaded configuration at least part of the stent may be substantially helically shaped. At least part of the longitudinal axis of the stent may be substantially helically shaped. In the loaded configuration at least part of the stent may be substantially spiral shaped. In the unloaded configuration at least part of the longitudinal axis of the stent may be substantially straight. Preferably in the unloaded configuration at least part of the stent is substantially cylindrically shaped. In the unloaded configuration at least part of the longitudinal axis of the stent may be curved in a two-dimensional plane.

The stent may be configured to move from the unloaded configuration to the loaded configuration upon application of a compressive load to the stent.

In one embodiment of the invention the stent comprises a plurality of annular elements. Preferably the stent comprises a plurality of primary connecting elements to connect adjacent annular elements. Ideally the stent comprises a first primary connecting element to connect a first annular element to a second annular element, and a second primary connecting element to connect the second annular element to a third annular element, the first primary connecting element being circumferentially offset from the second primary connecting element. This arrangement facilitates the three-dimensional curved shape of the stent in the loaded configuration. Most preferably in the unloaded configuration at least part of the longitudinal axis of the aggregation of the plurality of primary connecting elements is curved in three-dimensional space. Similarly this arrangement facilitates the three-dimensional curved shape of the stent in the loaded configuration.

In one example the first and second primary connecting elements extend generally perpendicularly to the circumferential direction, and join the second annular element at respective locations which are circumferentially offset. In other embodiments, the first and second primary connecting elements extend in a direction with a component in the circumferential direction and a component in the longitudinal direction. This arrangement can achieve the circumferential offset in an example where the first and second primary connecting elements join the second annular element at respective locations which are not circumferentially offset. In another example of the arrangement in which the first and second primary connecting elements extend in a direction with a component in the circumferential direction and a component in the longitudinal direction, the first and second primary connecting elements join the second annular element at respective locations which are circumferentially offset.

The stent may comprise a plurality of secondary connecting elements to connect adjacent annular elements. Preferably the stent comprises a first secondary connecting element to connect a first annular element to a second annular element, and a second secondary connecting element to connect the second annular element to a third annular element, the first secondary connecting element being circumferentially offset from the second secondary connecting element. This arrangement facilitates the three-dimensional curved shape of the stent in the loaded configuration. Ideally in the unloaded configuration at least part of the longitudinal axis of the aggregation of the plurality of secondary connecting elements is curved in three-dimensional space. Similarly this arrangement facilitates the three-dimensional curved shape of the stent in the loaded configuration.

The circumferential dimension of the primary connecting element may be greater than the circumferential dimension of the secondary connecting element. The longitudinal dimension of the primary connecting element may be greater than the longitudinal dimension of the secondary connecting element. The radial dimension of the primary connecting element may be greater than the radial dimension of the secondary connecting element. The stiffness of the primary connecting element may be less than the stiffness of the secondary connecting element.

In one case the longitudinal dimension of the annular element varies around the circumference of the annular element. Preferably the stent comprises a first annular element and a second annular element, the point on the circumference of the first annular element where the longitudinal dimension is at a maximum being circumferentially offset from the point on the circumference of the second annular element where the longitudinal dimension is at a maximum. This arrangement facilitates the three-dimensional curved shape of the stent in the loaded configuration. Ideally in the unloaded configuration at least part of the longitudinal axis of the aggregation of the plurality of points on the circumference of the annular elements where the longitudinal dimension is at a maximum is curved in three-dimensional space. Similarly this arrangement facilitates the three-dimensional curved shape of the stent in the loaded configuration.

In one case the stent comprises less than six connecting elements to connect a first annular element to a second annular element. By using a relatively small number of connecting elements, the stent is more readily able to move from the unloaded configuration to the loaded configuration with the three-dimensional curved shape. The stent may comprise less than four connecting elements to connect a first annular element to a second annular element. The stent may comprise a single connecting element to connect a first annular element to a second annular element.

The stent is suitable for location in a blood vessel. In the case of some blood vessels, for example the superficial femoral artery, upon application of a load to the blood vessel, for example as a result of bending a person's joint such as a knee or elbow, the blood vessel may curve in three-dimensional space. Because the stent located in the blood vessel is curved in three-dimensional space in the loaded configuration, this arrangement enables the stent to accommodate the blood vessel deformations in a controlled way. The stent comprises a stent suitable for deployment in a blood vessel. When the stent is deployed in the blood vessel, the stent exerts force on the blood vessel causing at least part of the longitudinal axis of the blood vessel to curve in three-dimensional space. Blood flowing through the three-dimensional curved part of the blood vessel undergoes a swirling action. The swirling flow of blood has been found to minimise thrombosis and platelet adhesion, and to minimise or prevent coverage of the stent by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia.

The stent is biased to achieve the three dimensional curvature during loading. Thus it may have properties which cause it to adopt the three-dimensional curvature during loading. The bias is built into the stent such that it will adopt a predetennined three-dimensional curvature in response to loading. The bias (or pre-set shape) of the stent is predetennined. This can be achieved by manufacturing the stent with certain properties. Examples of these properties are demonstrated by the preferred embodiments disclosed herein.

Considering a preferred stent, when in the loaded configuration, the stent then exerts force on the blood vessel causing at least part of the longitudinal axis of the blood vessel to curve in three-dimensional space. The stent may thus impose its own three-dimensional curvature on the vessel, rather than adopting the curvature of the vessel. The loading of the stent to its loaded configuration may be caused by deformation of the blood vessel, e.g. bending of the vessel. The deformation may cause an axial compressive load to be applied to the stent, and hence axial shortening.

The stent may be collapsed to a delivery configuration and inserted into a blood vessel. The stent may be advanced through the blood vessel using a delivery catheter to a deployment site. The stent may be caused to expand from the delivery configuration to a deployment configuration at the deployment site. The stent may be a self expanding stent, or alternatively the stent may be expanded using a balloon on the delivery catheter.

The deployed stent in the unloaded configuration may have a longitudinal axis at least part of which is substantially straight, or at least part of which is curved in a two-dimensional plane. Upon application of a load to the deployed stent, for example a bending load or an axially compressive load, which may be caused by bending of the vessel, the stent moves from the unloaded configuration to the loaded configuration. The deployed stent in the loaded configuration has a three-dimensional curved longitudinal axis, e.g. a helically shaped axis.

The inventors have recognised that bending of a body lumen will in many instances give rise to axial compression of the stent in the lumen. They devised a stent which when axially compressed to a loaded configuration adopts a shape, in which at least part of the longitudinal axis of the stent is curved in three-dimensional space.

In a method disclosed herein, a stent which is deployed in its unloaded configuration is bent and hence axially compressed by a body lumen and moves from the unloaded configuration to the loaded configuration. The body lumen may for example be the superficial femoral artery.

### Brief Description of the Drawings

The invention will be more clearly understood from the following description of some embodiments thereof, given by way of example only, with reference to the accompanying drawings, in which:
Fig. 1 is an isometric view of a stent according to the invention in an unloaded configuration;
Fig. 2 is an isometric view of the stent of Fig. 1 in a loaded configuration;
Fig. 3 is an isometric view of another stent according to the invention in an unloaded configuration;
Fig. 4 is an isometric view of the stent of Fig. 3 in a loaded configuration;
Fig. 5 is an isometric view of another stent according to the invention in an unloaded configuration;
Fig. 6 is an enlarged isometric view of part of the stent of Fig. 5 in the unloaded configuration;
Fig. 7 is an isometric view of the stent of Fig. 5 in a loaded configuration;
Fig. 8 is an isometric view of another stent according to the invention in an unloaded configuration;
Fig. 9 is an isometric view of the stent of Fig. 8 in a loaded configuration;
Fig. 10 is an isometric view of another stent according to the invention in an unloaded configuration;
Fig. 11 is an isometric view of the stent of Fig. 10 in a loaded configuration;
Fig. 12 is a front view of the stent of Fig. 10 in the unloaded configuration;
Fig. 13 is an end view of the stent of Fig. 10 in the unloaded configuration;
Fig. 14 is a front view of the stent of Fig. 10 in the loaded configuration;
Fig. 15 is an end view of the stent of Fig. 10 in the loaded configuration;
Fig. 16 is an isometric view of another stent according to the invention in an unloaded configuration;
Fig. 17 is an enlarged isometric view of part of the stent of Fig. 16 in the unloaded configuration; and
Fig. 18 is an isometric view of the stent of Fig. 16 in a loaded configuration.

### Detailed Description

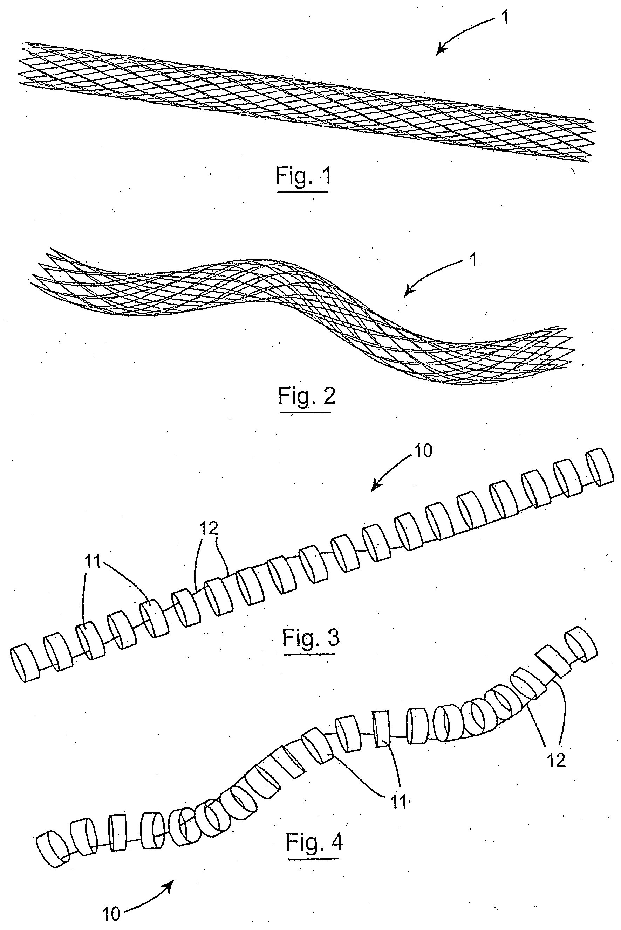

Referring to the drawings, and initially to Figs. 1 and 2 thereof, there is illustrated a stent 1 according to the invention suitable for deployment in a blood vessel to support at least part of an internal wall of the blood vessel.

Upon application of a load to the stent 1, such as a compressive load, the stent 1 moves from an unloaded configuration (Fig. 1) to a loaded configuration (Fig. 2). In the unloaded configuration the longitudinal axis of the stent 1 is straight, and the stent 1 is cylindrically shaped. In the loaded configuration the longitudinal axis of the stent 1 is curved in three-dimensional space, and the stent 1 is helically shaped.

Figs. 1 and 2 illustrate the unloaded and loaded configurations of the stent 1 which is biased to achieve the three dimensional curvature during loading.

It will be appreciated that the stent 1 may have an alternative shape in the loaded configuration, for example a spiral shape.

In use, the stent 1 is collapsed to a delivery configuration and inserted into the blood vessel. The stent 1 is advanced through the blood vessel using a delivery catheter to a deployment site. The stent 1 is caused to expand from the delivery configuration to a deployment configuration at the deployment site. The stent 1 may be a self expanding stent, or alternatively the stent 1 may be expanded using a balloon on the delivery catheter.

The deployed stent 1 in the unloaded configuration is cylindrically shaped with the straight longitudinal axis. Upon application of a load to the stent 1, the stent 1 moves from the unloaded configuration to the loaded configuration. The deployed stent 1 in the loaded configuration is helically shaped with the three-dimensional curved longitudinal axis.

The stent 1 has the straight configuration outside of the blood vessel, and the straight configuration inside the blood vessel before loading. When loaded the stent 1 moves into the three dimensional curvature configuration, such as helical or spiral. The stent 1 is biased to achieve the three dimensional curvature during loading. This controlled change in geometry may be achieved through regional variations in the properties of the stent 1.

The deployment site for the stent 1 may be in the blood vessel in the leg behind the knee which is subject to frequent bending as the patient bends the leg. During bending of the leg, the path of this blood vessel which is known as the superficial femoral artery may shorten, causing its geometry to change from straight into a spiral configuration. This results in the artery having a three dimensional curvature when loaded. The deployed stent 1 in the superficial femoral artery is also subject to this compressive loading. Because the stent 1 is biased into taking up a three dimensional configuration within the artery during loading, the stent 1 accommodates the blood vessel deformations in a controlled way, improving fracture resistance while also improving flow conditions.

When the stent 1 is deployed in the blood vessel and is caused to adopt the loaded configuration, the stent 1 exerts force on the blood vessel causing at least part of the longitudinal axis of the blood vessel to curve in three-dimensional space. In this manner the stent 1 acts to support at least part of the internal wall of the blood vessel curved in three-dimensional space. Blood flowing through the three-dimensional curved part of the blood vessel then undergoes a swirling action. The swirling flow of blood has been found to minimise thrombosis and platelet adhesion, and to minimise or prevent coverage of the stent 1 by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia.

The helical shape of the loaded stent 1 ensures that there are no points of high stress in the stent 1.

In Figs. 3 and 4 there is illustrated another stent 10 according to the invention, which is similar to the stent 1 of Figs. 1 and 2.

In this case the stent 10 comprises a plurality of longitudinally spaced-apart annular elements 11, and a plurality of primary connecting elements 12 to connect adjacent annular elements 11. A single connecting element 12 connects each annular element 11 to an adjacent annular element 11. The annular elements 11 are shown schematically as solid cylinders but in practice may be made up of struts or the like, or a combination of struts or the like and a covering material, together creating an overall annular shape.

Each primary connecting element 12 is circumferentially offset from the previous primary connecting element 12. In the unloaded configuration (Fig. 3) the longitudinal axis of the aggregation of the plurality of primary connecting elements 12 is curved in three-dimensional space. In this manner the connecting elements 12 snake helically around the stent 10.

In this patent specification the term 'aggregation' will be understood to mean the grouping together of separate elements so that the separate elements may be considered as a whole.

Figs. 3 and 4 illustrate the stent 10 in which the location of the axial connecting elements 12 which connect the annular elements 11 dictate the loaded, deformed geometry of the stent 10. Since the connecting elements 12 are offset from one another in subsequent annular elements 11, the stent 10 will take up the three dimensional, e.g. helical, centreline curvature upon compressive loading.

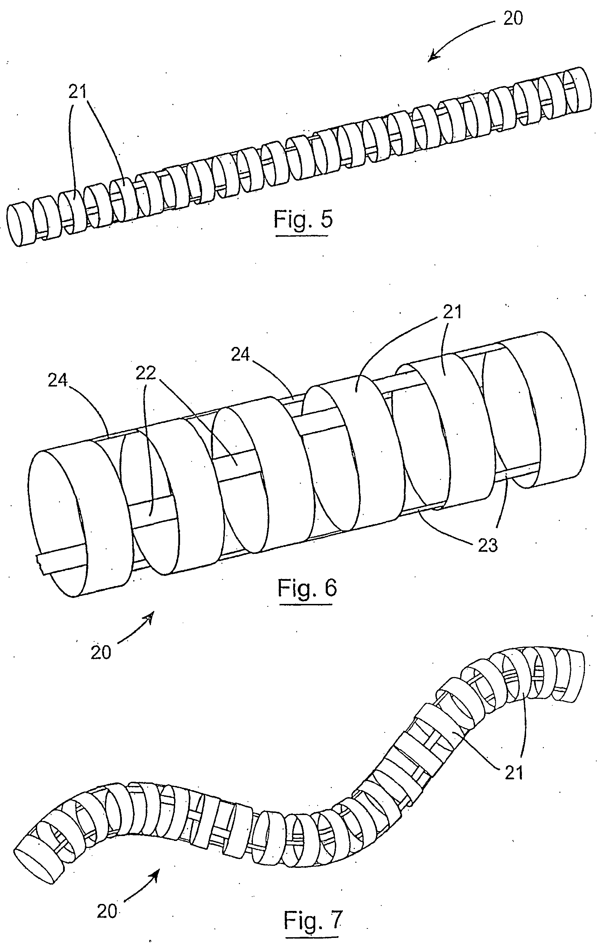

Figs. 5 to 7 illustrate a further stent 20 according to the invention, which is similar to the stent 10 of Figs. 3 and 4.

In this case the stent 20 comprises the plurality of longitudinally spaced-apart annular elements 21, the plurality of primary connecting elements 22 to connect adjacent annular elements 21, a plurality of secondary connecting elements 23 to connect adjacent annular elements 21, and a plurality of tertiary connecting elements 24 to connect adjacent annular elements 21. Three connecting elements 22, 23, 24 connect each annular element 21 to an adjacent annular element 21. The annular elements 21 are shown schematically as solid cylinders but in practice may be made up of struts or the like, or a combination of struts or the like and a covering material, together creating an overall annular shape.

Each primary connecting element 22 is circumferentially offset from the previous primary connecting element 22. In the unloaded configuration (Figs. 5 and 6) the longitudinal axis of the aggregation of the plurality of primary connecting elements 22 is curved in three-dimensional space. Similarly each secondary connecting element 23 is circumferentially offset from the previous secondary connecting element 23. In the unloaded configuration (Figs. 5 and 6) the longitudinal axis of the aggregation of the plurality of secondary connecting elements 23 is curved in three-dimensional space. Similarly each tertiary connecting element 24 is circumferentially offset from the previous tertiary connecting element 24. In the unloaded configuration (Figs. 5 and 6) the longitudinal axis of the aggregation of the plurality of tertiary connecting elements 24 is curved in three-dimensional space.

The circumferential dimension of each primary connecting element 22 is greater than the circumferential dimension of each secondary connecting element 23, and the circumferential dimension of each primary connecting element 22 is greater than the circumferential dimension of each tertiary connecting element 24. In this case the circumferential dimension of each secondary connecting element 23 is equal to the circumferential dimension of each tertiary connecting element 24. The dominant connecting element 22 is of larger dimension than the other connecting elements 23, 24. The dominant connecting element 22 is arranged in the helical snake form.

Figs. 5 to 7 illustrate the variations in the strut thickness used to achieve the three dimensional curvature during loading. The stent 20 has the struts 22, 23, 24 of differing width between the annular elements 21. The strut width pattern is indexed by an angle between adjacent annular elements 21. Under a compressive load the stent 20 will compress into a helical shape with three dimensional longitudinal centreline curvature.

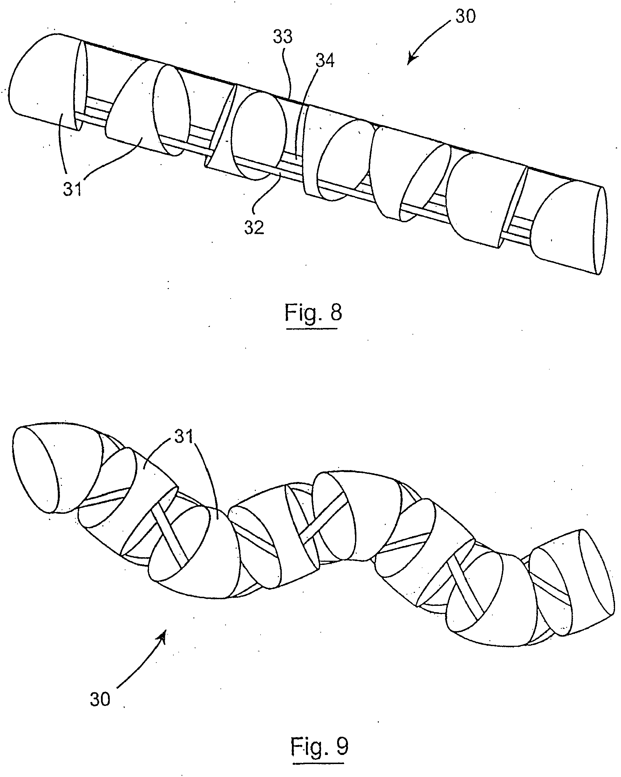

Referring to Figs. 8 and 9 there is illustrated another stent 30 according to the invention, which is similar to the stent 20 of Figs. 5 to 7.

In this case the stent 30 comprises the plurality of longitudinally spaced-apart annular elements 31, the plurality of primary connecting elements 32 to connect adjacent annular elements 31, the plurality of secondary connecting elements 33 to connect adjacent annular elements 31, and the plurality of tertiary connecting elements 34 to connect adjacent annular elements 31. Three connecting elements 32, 33, 34 connect each annular element 31 to an adjacent annular element 31. The annular elements 31 are shown schematically as solid cylinders but in practice may be made up of struts or the like, or a combination of struts or the like and a covering material, together creating an overall annular shape.

The longitudinal dimension of each annular element 31 varies around the circumference of the annular element 31. For each annular element 31 the point on the circumference of the annular element 31 where the longitudinal dimension is at a maximum is circumferentially offset from the point on the circumference of the previous annular element 31 where the longitudinal dimension is at a maximum. In the unloaded configuration (Fig. 8) the longitudinal axis of the aggregation of the points of maximum longitudinal dimension is curved in three-dimensional space, e.g. helical.

Each primary connecting element 32 is circumferentially offset from the previous primary connecting element 32. In the unloaded configuration (Fig. 8) the longitudinal axis of the aggregation of the plurality of primary connecting elements 32 is curved in three-dimensional space, e.g. helical. Similarly each secondary connecting element 33 is circumferentially offset from the previous secondary connecting element 33. In the unloaded configuration (Fig. 8) the longitudinal axis of the aggregation of the plurality of secondary connecting elements 33 is curved in three-dimensional space, e.g. helical. Similarly each tertiary connecting element 34 is circumferentially offset from the previous tertiary connecting element 34. In the unloaded configuration (Fig. 8) the longitudinal axis of the aggregation of the plurality of tertiary connecting elements 34 is curved in three-dimensional space, e.g. helical.

The longitudinal dimension of each primary connecting element 32 is greater than the longitudinal dimension of each secondary connecting element 33, and the longitudinal dimension of each secondary connecting element 33 is greater than the longitudinal dimension of each tertiary connecting element 34.

In Figs. 8 and 9 each annular element 31 has a variable length. Subsequent annular elements 31 are rotated relative to each other and this results in the connectors 32, 33, 34 between the annular elements 31 having variable lengths. The short side of the annular elements 31 are arranged in a spiral fashion, such that under a compressive load the stent 30 will compress into a helical shape with three dimensional longitudinal centreline curvature.

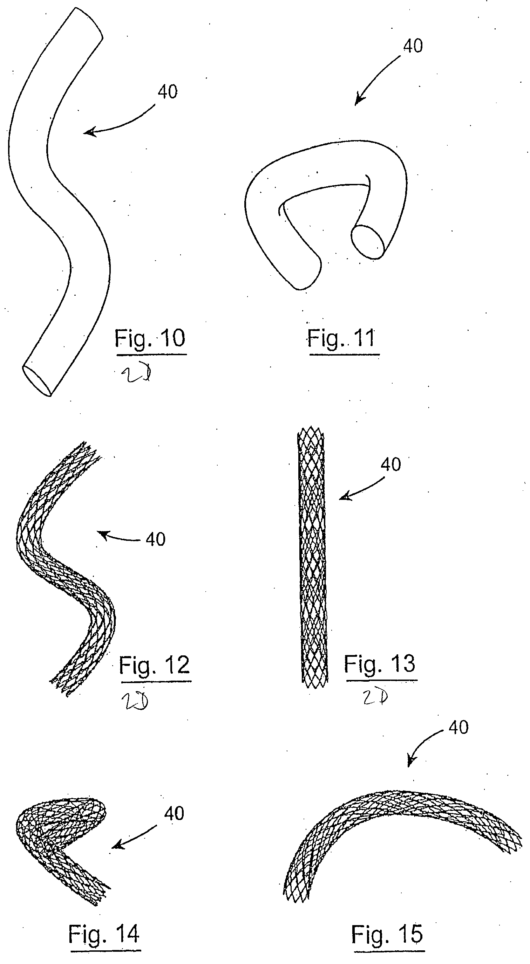

In Figs. 10 to 15 there is illustrated another stent 40 according to the invention, which is similar to the stent 1 of Figs. 1 and 2.

In this case the longitudinal axis of the stent 40 is curved in a two-dimensional plane in the unloaded configuration (Figs. 10, 12, 13).

Figs. 10 to 15 illustrate the stent 40 with the two-dimensional bend when in the unloaded state. Bending of the stent 40, for example behind the knee, induces the three-dimensional curvature. When implanted at a site subject to bending, for example behind the knee, three dimensional curvature is achieved when the stent 40 bends.

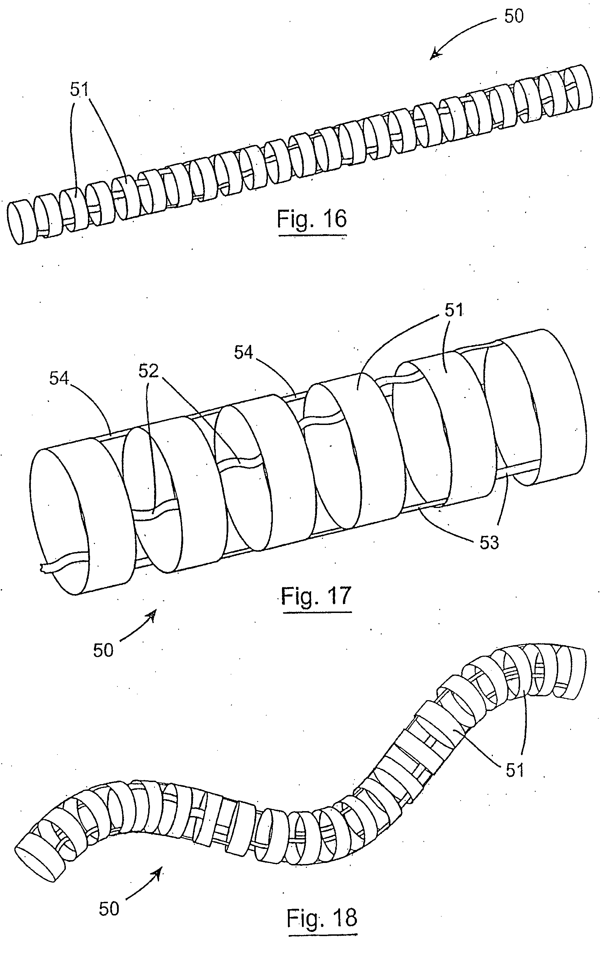

Figs. 16 to 18 illustrate a further stent 50 according to the invention, which is similar to the stent 20 of Figs. 5 to 7.

In this case the stent 50 comprises the plurality of longitudinally spaced-apart annular elements 51, the plurality of primary connecting elements 52 to connect adjacent annular elements 51, the plurality of secondary connecting elements 53 to connect adjacent annular elements 51, and the plurality of tertiary connecting elements 54 to connect adjacent annular elements 51. Three connecting elements 52, 53, 54 connect each annular element 51 to an adjacent annular element 51. The annular elements 51 are shown schematically as solid cylinders but in practice may be made up of struts or the like, or a combination of struts or the like and a covering material, together creating an overall annular shape.

Each primary connecting element 52 is circumferentially offset from the previous primary connecting element 52. In the unloaded configuration (Figs. 16 and 17) the longitudinal axis of the aggregation of the plurality of primary connecting elements 52 is curved in three-dimensional space, e.g. helical. Similarly each secondary connecting element 53 is circumferentially offset from the previous secondary connecting element 53. In the unloaded configuration (Figs. 16 and 17) the longitudinal axis of the aggregation of the plurality of secondary connecting elements 53 is curved in three-dimensional space, e.g. helical. Similarly each tertiary connecting element 54 is circumferentially offset from the previous tertiary connecting element 54. In the unloaded configuration (Figs. 16 and 17) the longitudinal axis of the aggregation of the plurality of tertiary connecting elements 54 is curved in three-dimensional space, e.g. helical.

The circumferential dimension of each primary connecting element 52 is equal to the circumferential dimension of each secondary connecting element 53, and the circumferential dimension of each primary connecting element 52 is equal to the circumferential dimension of each tertiary connecting element 54.

Each primary connecting element 52 extends in a curve between adjacent annular elements 51 to form a point of weakness in the primary connecting element 52. Each secondary connecting element 53 and each tertiary connecting element 54 extends in a straight manner between adjacent annular elements 51. In this manner the stiffness of each primary connecting element 52 is less than the stiffness of each secondary connecting element 53, and the stiffness of each primary connecting element 52 is less than the stiffness of each tertiary connecting element 54. Under a compressive load the stent 50 will compress into a helical shape with three dimensional longitudinal centreline curvature.

It will be appreciated that the three-dimensional curved shape of the stent in the loaded configuration may be achieved using a variety of possible means.

The radial dimension of the primary connecting element may be greater than the radial dimension of the secondary connecting element.

The three dimensional curvature may be achieved by varying the width and/or thickness of the axial connecting elements between adjacent annular elements.

The stent may include a point of weakness. The point of weakness may be arranged in the helical snake form.

The number of connecting elements may be varied, the geometry of the annular elements may be varied, the overall stent pattern may be varied.

Regional variations in the properties of the stent may be achieved by varying the materials used to construct the stent.

Any combination of the means disclosed may be used to achieve the desired regional variations in the stent properties, which leads to the three dimensional curvature during loading.

It will also be appreciated that the stent may be of any suitable construction, for example the stent may be of braided construction, the stent may be cut from a tube as a self expanding or balloon expandable stent, the stent may comprise elements connected using welds. Each annular element may be in the form of a crown or alternatively may be in the form of a cuff which extends circumferentially in a hoop. The stent may be a stent graft.

It will further be appreciated that although compression is given as an example of loading, the stent may achieve the three dimensional curvature under other loading modes depending on the regional property variations in the stent.

It will also be appreciated that the stent of the invention is suitable for location in a variety of types of blood vessel, for example the stent may be employed in a coronary application.

The invention is not limited to the embodiments hereinbefore described, with reference to the accompanying drawings, which may be varied in construction and detail.

## Claims

1. A stent (1, 10, 20, 30, 40 , 50) suitable for deployment in a blood vessel, wherein the stent is expandable from a delivery configuration to a deployment configuration, wherein upon application of a load to the stent when the stent is in the deployment configuration the stent is movable from an unloaded configuration to a loaded configuration, and wherein in the loaded configuration at least part of the longitudinal axis of the stent is curved in three-dimensional space, **characterised in that** in the unloaded configuration said part of the longitudinal axis of the stent is substantially straight or is curved in a two-dimensional plane, the stent being biased to achieve the three-dimensional curvature during loading.

2. A stent as claimed in claim 1 wherein in the loaded configuration at least part of the stent is substantially helically shaped, or wherein in the loaded configuration at least part of the stent is substantially spiral shaped.

3. A stent as claimed in claim 1 or 2 wherein in the unloaded configuration at least part of the longitudinal axis of the stent is substantially straight, and wherein in the unloaded configuration at least part of the stent is substantially cylindrically shaped.

4. A stent as claimed in any of claims 1 to 3 wherein the stent is configured to move from the unloaded configuration to the loaded configuration upon application of a compressive load to the stent.

5. A stent as claimed in any of claims 1 to 4 wherein the stent comprises a plurality of annular elements(11, 21, 31 ,51), preferably wherein the stent comprises a plurality of primary connecting elements(12, 22, 32, 52) to connect adjacent annular elements, more preferably wherein the stent comprises a first primary connecting element to connect a first annular element to a second annular element, and a second primary connecting element to connect the second annular element to a third annular element, the first primary connecting element being circumferentially offset from the second primary connecting element.

6. A stent as claimed in claim 5 wherein in the unloaded configuration at least part of the longitudinal axis of the aggregation of the plurality of primary connecting elements is curved in three-dimensional space.

7. A stent as claimed in claim 5 or 6 wherein the stent comprises a plurality of secondary connecting elements (23, 33, 53) to connect adjacent annular elements, preferably wherein the stent comprises a first secondary connecting element to connect a first annular element to a second annular element, and a second secondary connecting element to connect the second annular element to a third annular element, the first secondary connecting element being circumferentially offset from the second secondary connecting element.

8. A stent as claimed in claim 7 wherein in the unloaded configuration at least part of the longitudinal axis of the aggregation of the plurality of secondary connecting elements is curved in three-dimensional space.

9. A stent as claimed in claim 7 or 8 wherein the circumferential dimension of the primary connecting element is greater than the circumferential dimension of the secondary connecting element.

10. A stent as claimed in any of claims 7 to 9 wherein the longitudinal dimension of the primary connecting element is greater than the longitudinal dimension of the secondary connecting element.

11. A stent as claimed in any of claims 7 to 10 wherein the radial dimension of the primary connecting element is greater than the radial dimension of the secondary connecting element.

12. A stent as claimed in any of claims 7 to 11 wherein the stiffness of the primary connecting element is less than the stiffness of the secondary connecting element.

13. A stent as claimed in any of claims 5 to 12 wherein the longitudinal dimension of the annular element varies around the circumference of the annular element, preferably wherein the stent comprises a first annular element and a second annular element, the point on the circumference of the first annular element where the longitudinal dimension is at a maximum being circumferentially offset from the point on the circumference of the second annular element where the longitudinal dimension is at a maximum.

14. A stent as claimed in claim 13 wherein in the unloaded configuration at least part of the longitudinal axis of the aggregation of the plurality of points on the circumference of the annular elements where the longitudinal dimension is at a maximum is curved in three-dimensional space.

15. A stent as claimed in any of claims 5 to 14 wherein the stent comprises less than six connecting elements to connect a first annular element to a second annular element, preferably wherein the stent comprises less than four connecting elements to connect a first annular element to a second annular element, more preferably wherein the stent comprises a single connecting element to connect a first annular element to a second annular element.

## Patentansprüche

1. Stent (1, 10, 20, 30, 40, 50), welcher für eine Entfaltung in einem Blutgefäß geeignet ist, wobei der Stent von einer Einführauslegung in eine Entfaltungsauslegung expandierbar ist, wobei beim Ausüben einer Belastung auf den Stent, wenn sich der Stent in der Entfaltungsauslegung befindet, der Stent von einer unbelasteten Auslegung in eine belastete Auslegung bewegbar ist, und wobei in der belasteten Auslegung wenigstens ein Teil der Längsachse des Stents im dreidimensionalen Raum gekrümmt ist, **dadurch gekennzeichnet, dass** in der unbelasteten Auslegung der Teil der Längsachse des Stents im Wesentlichen gerade ist oder in einer zweidimensionalen Ebene gekrümmt ist, wobei der Stent vorgespannt ist, um die dreidimensionale Krümmung während der Belastung zu erzielen.

2. Stent nach Anspruch 1, wobei in der belasteten Auslegung wenigstens ein Teil des Stents im Wesentlichen helixförmig ist, oder wobei in der belasteten Auslegung wenigstens ein Teil des Stents im Wesentlichen spiralförmig ist.

3. Stent nach Anspruch 1 oder 2, wobei in der unbelasteten Auslegung wenigstens ein Teil der Längsachse des Stents im Wesentlichen gerade ist, und wobei in der unbelasteten Auslegung wenigstens ein Teil des Stents im Wesentlichen zylinderförmig ist.

4. Stent nach einem der Ansprüche 1 bis 3, wobei der Stent ausgelegt ist, sich beim Ausüben einer Druckkraft auf den Stent von der unbelasteten Auslegung in die belastete Auslegung zu bewegen.

5. Stent nach einem der Ansprüche 1 bis 4, wobei der Stent mehrere ringförmige Elemente (11, 21, 31, 51) umfasst, wobei der Stent vorzugsweise mehrere primäre Verbindungselemente (12, 22, 32, 52) umfasst, um benachbarte ringförmige Elemente zu verbinden, wobei der Stent insbesondere ein erstes primäres Verbindungselement umfasst, um ein erstes ringförmiges Element mit einem zweiten ringförmigen Element zu verbinden, und ein zweites primäres Verbindungselement, um das zweite ringförmige Element mit einem dritten ringförmigen Element zu verbinden, welches erste primäre Verbindungselement in Umfangsrichtung vom zweiten primären Verbindungselement versetzt ist.

6. Stent nach Anspruch 5, wobei in der unbelasteten Auslegung wenigstens ein Teil der Längsachse der Ansammlung der mehreren primären Verbindungselemente im dreidimensionalen Raum gekrümmt ist.

7. Stent nach Anspruch 5 oder 6, wobei der Stent mehrere sekundäre Verbindungselemente (23, 33, 53) umfasst, um benachbarte ringförmige Elemente zu verbinden, wobei der Stent vorzugsweise ein erstes sekundäres Verbindungselement umfasst, um ein erstes ringförmiges Element mit einem zweiten ringförmigen Element zu verbinden, und ein zweites sekundäres Verbindungselement, um das zweite ringförmige Element mit einem dritten ringförmigen Element zu verbinden, welches erste sekundäre Verbindungselement in Umfangsrichtung vom zweiten sekundären Verbindungselement versetzt ist.

8. Stent nach Anspruch 7, wobei in der unbelasteten Auslegung wenigstens ein Teil der Längsachse der Ansammlung der mehreren sekundären Verbindungselemente im dreidimensionalen Raum gekrümmt ist.

9. Stent nach Anspruch 7 oder 8, wobei die Umfangsabmessung des primären Verbindungselements größer ist als die Umfangsabmessung des sekundären Verbindungselements.

10. Stent nach einem der Ansprüche 7 bis 9, wobei die Längsabmessung des primären Verbindungselements größer ist als die Längsabmessung des sekundären Verbindungselements.

11. Stent nach einem der Ansprüche 7 bis 10, wobei die radiale Abmessung des primären Verbindungselements größer ist als die radiale Abmessung des sekundären Verbindungselements.

12. Stent nach einem der Ansprüche 7 bis 11, wobei die Steifigkeit des primären Verbindungselements geringer ist als die Steifigkeit des sekundären Verbindungselements.

13. Stent nach einem der Ansprüche 5 bis 12, wobei die Längsabmessung des ringförmigen Elements rund um den Umfang des ringförmigen Elements variiert, wobei der Stent vorzugsweise ein erstes ringförmiges Element und ein zweites ringförmiges Element umfasst, wobei der Punkt am Umfang des ersten ringförmigen Elements, wo die Längsabmessung auf einem Maximum ist, in Umfangsrichtung von dem Punkt am Umfang des zweiten ringförmigen Elements versetzt ist, wo die Längsabmessung auf einem Maximum ist.

14. Stent nach Anspruch 13, wobei in der unbelasteten Auslegung wenigstens ein Teil der Längsachse der Ansammlung der mehreren Punkte am Umfang der ringförmigen Elemente, wo die Längsabmessung auf einem Maximum ist, im dreidimensionalen Raum gekrümmt ist.

15. Stent nach einem der Ansprüche 5 bis 14, wobei der Stent weniger als sechs Verbindungselemente umfasst, um ein erstes ringförmiges Element mit einem zweiten ringförmigen Element zu verbinden, wobei der Stent vorzugsweise weniger als vier Verbindungselemente umfasst, um ein erstes ringförmiges Element mit einem zweiten ringförmigen Element zu verbinden, wobei der Stent insbesondere ein einzelnes Verbindungselement umfasst, um ein erstes ringförmiges Element mit einem zweiten ringförmigen Element zu verbinden.

## Revendications

1. Stent (1, 10, 20, 30, 40, 50) convenant à un déploiement dans un vaisseau sanguin, dans lequel le stent peut se dilater d'une configuration de délivrance à une configuration de déploiement, dans lequel, lors de l'application d'une charge au stent, lorsque le stent se trouve dans la configuration de déploiement, le stent peut se déplacer d'une configuration non chargée à une configuration chargée, et dans lequel, dans la configuration chargée, au moins une partie de l'axe longitudinal du stent est incurvée dans l'espace tridimensionnel, **caractérisé en ce que**, dans la configuration non chargée, ladite partie de l'axe longitudinal du stent est sensiblement droite ou est incurvée dans un plan bidimensionnel, le stent étant sollicité pour adopter la courbure tridimensionnelle au cours du chargement.

2. Stent selon la revendication 1, dans lequel, dans la configuration chargée, au moins une partie du stent est sensiblement conformée en hélice ou dans lequel, dans la configuration chargée, au moins une partie du stent est sensiblement conformée en spirale.

3. Stent selon la revendication 1 ou la revendication 2, dans lequel, dans la configuration non chargée, au moins une partie de l'axe longitudinal du stent est sensiblement droite et dans lequel, dans la configuration non chargée, au moins une partie du stent a une forme sensiblement cylindrique.

4. Stent selon l'une quelconque des revendications 1 à 3, dans lequel le stent est configuré pour se déplacer de la configuration non chargée à la configuration chargée par application d'une charge compressive au stent.

5. Stent selon l'une quelconque des revendications 1 à 4, dans lequel le stent comprend une pluralité d'éléments annulaires (11, 21, 31, 51), de préférence dans lequel le stent comprend une pluralité d'éléments de liaison primaires (12, 22, 32, 52) pour relier des éléments annulaires adjacents, mieux encore dans lequel le stent comprend un premier élément de liaison primaire pour relier un premier élément annulaire à un deuxième élément annulaire et un second élément de liaison primaire pour relier le second élément annulaire à un troisième élément annulaire, le premier élément de liaison primaire étant circonférentiellement décalé du deuxième élément de liaison primaire.

6. Stent selon la revendication 5, dans lequel, dans la configuration non chargée, au moins une partie de l'axe longitudinal de l'agrégation de la pluralité d'éléments de liaison primaires est incurvée dans l'espace tridimensionnel.

7. Stent selon la revendication 5 ou la revendication 6, dans lequel le stent comprend une pluralité d'éléments de liaison secondaires (23, 33, 53) pour relier des éléments annulaires adjacents, de préférence dans lequel le stent comprend un premier élément de liaison secondaire pour relier un premier élément annulaire à un deuxième élément annulaire et un second élément de liaison secondaire pour relier le deuxième élément annulaire à un troisième élément annulaire, le premier élément de liaison secondaire étant décalé circonférentiellement du deuxième élément de liaison secondaire.

8. Stent selon la revendication 7, dans lequel, dans la configuration non chargée, au moins une partie de l'axe longitudinal de l'agrégation de la pluralité d'éléments de liaison secondaires est incurvée dans l'espace tridimensionnel.

9. Stent selon la revendication 7 ou la revendication 8, dans lequel la dimension circonférentielle de l'élément de liaison primaire est supérieure à la dimension circonférentielle de l'élément de liaison secondaire.

10. Stent selon l'une quelconque des revendications 7 à 9, dans lequel la dimension longitudinale de l'élément de liaison primaire est supérieure à la dimension longitudinale de l'élément de liaison secondaire.

11. Stent selon l'une quelconque des revendications 7 à 10, dans lequel la dimension radiale de l'élément de liaison primaire est supérieure à la dimension radiale de l'élément de liaison secondaire.

12. Stent selon l'une quelconque des revendications 7 à 11, dans lequel la rigidité de l'élément de liaison primaire est inférieure à la rigidité de l'élément de liaison secondaire.

13. Stent selon l'une quelconque des revendications 5 à 12, dans lequel la dimension longitudinale de l'élément annulaire varie autour de la circonférence de l'élément annulaire, de préférence dans lequel le stent comprend un premier élément annulaire et un deuxième élément annulaire, le point sur la circonférence du premier élément annulaire où la dimension longitudinale est au maximum étant décalé circonférentiellement du point sur la circonférence du deuxième élément annulaire où la dimension longitudinale est au maximum.

14. Stent selon la revendication 13, dans lequel, dans la configuration non chargée, au moins une partie de l'axe longitudinal de l'agrégation de la pluralité de points sur la circonférence des éléments annulaires où la dimension longitudinale est au maximum est incurvée dans l'espace tridimensionnel.

15. Stent selon l'une quelconque des revendications 5 à 14, dans lequel le stent comprend moins de six éléments de liaison pour relier un premier élément annulaire à un deuxième élément annulaire, de préférence dans lequel le stent comprend moins de quatre éléments de liaison pour relier un premier élément annulaire à un deuxième élément annulaire, mieux encore dans lequel le stent comprend un seul élément de liaison pour relier un premier élément annulaire à un deuxième élément annulaire.
